# EUROPÄISCHE PATENTSCHRIFT

(11) **EP 0 521 004 B1**
(45) Veröffentlichungstag und Bekanntmachung des Hinweises auf die Patenterteilung: **05.04.1995**
(21) Anmeldenummer: 91904811.6
(22) Anmeldetag: 02.03.1991
(51) Int. Cl.: A61K 7/32

(54) **DESODORIERENDE KOSMETISCHE MITTEL**
COSMETIC DEODORANTS
AGENTS COSMETIQUES DESODORISANTS

(30) Priorität: 23.03.1990 DE 4009347
(43) Veröffentlichungstag der Anmeldung: 07.01.1993
(73) Patentinhaber: Beiersdorf Aktiengesellschaft, 20245 Hamburg (DE)
(72) Erfinder: KLEIN, Winfried, D-2000 Hamburg 60 (DE); KADEN, Waltraud, D-2083 Halstenbek (DE); RÖCKL, Manfred, D-2000 Wedel (DE)
(86) Internationale Anmeldenummer: DE9100189
(87) Internationale Veröffentlichungsnummer: WO9114419

(56) Entgegenhaltungen:
- EP-A- 0 203 681
- DE-A- 2 513 347
- FR-A- 688 181
- FR-A- 1 199 349
- FR-A- 2 198 728
- Patent Abstracts of Japan, Band 14, Nr. 77 (C-688)(4020), 14. Februar 1990; &JP-A-1294615 (SHISEIDO CO. LTD) 28. November 1989
- STN Datenbank Liefferant (Karlsruhe, DE), Datenbank Chemical Abstracts, Band103, Nr. 27093q

## Beschreibung

Die vorliegende Erfindung betrifft kosmetische Desodorantien. Solche Formulierungen dienen dazu, Körpergeruch zu beseitigen, der entsteht, wenn der an sich geruchlose frische Schweiß durch Mikroorganismen zersetzt wird. Den handelsüblichen kosmetischen Desodorantien liegen unterschiedliche Wirkprinzipien zugrunde.

Durch Adstringentien - vorwiegend Aluminiumsalze wie Aluminiumhydroxychlorid - kann die Entstehung des Schweißes unterbunden werden. Abgesehen von der Denaturierung der Hautproteine greifen die dafür verwendeten Stoffe aber drastisch in den Wärmehaushalt der Achselregion ein und sollten allenfalls in Ausnahmefällen angewandt werden.

Durch antimikrobielle Stoffe kann die Bakterienflora auf der Haut reduziert werden. Dabei sollten im Idealfalle nur die Geruch verursachenden Mikroorganismen vernichtet werden. In der Praxis hat sich aber herausgestellt, daß die gesamte Mikroflora der Haut in gleichem Maße geschädigt werden. Gelegentlich werden sogar die Mikroorganismen, die keinen Geruch verursachen, stärker geschädigt.

Schließlich kann Körpergeruch auch durch Duftstoffe überdeckt werden. Dies ist die klassische Methode, Sie wird aber am wenigsten den ästhetischen Bedürfnissen des Verbrauchers gerecht: Die Mischung aus Körpergeruch und Parfümduft riecht eher penetrant als angenehm.

Desodorantien sollen folgende Bedingungen erfüllen:
1) Die biologischen Vorgänge der Haut dürfen nicht beeinträchtigt werden
2) Die Desodorantien sollen keinen ausgeprägten Eigengeruch besitzen
3) Sie müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in handelsübliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays usw.

Aufgabe der vorliegenden Erfindung war es, kosmetische Desodorantien zu entwickeln, die die Nachteile des Standes der Technik nicht aufweisen. Insbesondere sollten die Desodorantien die Mikroflora der Haut weitgehend schonen, die Mikroorganismen aber, die für den Körpergeruch verantwortlich sind, selektiv reduzieren.

Es wurde gefunden, und darin liegt die Lösung der Aufgabe, daß kosmetische Desodorantien in Form von Suspensionen,
(a) enthaltend ein oder mehrere Schichtsilicate, gewählt aus der Gruppe
   Montmorillonit, Kaolinit, Ilit, Beidellit, Nontronit, Saponit, Hectorit, Bentonit, Smectit,
(b) wobei der Wassergehalt der kosmetischen Desodorantien 5 Gew.-% nicht übersteigt,
(c) wobei der Ethanolgehalt der kosmetischen Desodorantien 35 Gew.-% nicht übersteigt,
den Nachteilen des Standes der Technik abhelfen.

Zwar beschreibt FR-A-2 198 728 desodorierende kosmetische Zubereitungen mit einem Gehalt an Schichtsilikaten. Der Wassergehalt der beschriebenen Zubereitungen ist jedoch viel zu hoch, als daß die erfindungsgemäße Wirkung hätte erzielt werden können. Diese Schrift weist also nicht in die Richtung der vorliegenden Erfindung.

In den Chemical Abstracts, Band 103, Nr.27093q werden desodorierende Mittel für Haustiere mit einem Gehalt an Montmorillonit beschrieben. Weder liegen diese Mittel als Suspensionen vor, noch betrifft die Verbindung kosmetische Desodorantien zur Bekämpfung des Schweißgeruches.

Es ist günstig, reine Komponenten einzusetzen. Besonders vorteilhaft ist, Gemische verschiedener Schichtsilicate, wie sie etwa in natürlichen Mineralien vorkommen, den erfindungsgemäßen Zusammensetzungen einzuverleiben.

Unter Schichtsilicaten, welche auch Phyllosilicate genannt werden, sind im Rahmen dieser Anmeldung Silicate und Alumosilicate zu verstehen, in welchen die Silicat- bzw. Aluminateinheiten über drei Si-O- oder Al-O- Bindungen untereinander verknüpft sind und eine gewellte Blatt- oder Schichtenstruktur ausbilden. Die vierte Si-O- bzw. Al-O-Valenz wird durch Kationen abgesättigt. Zwischen den einzelnen Schichten bestehen schwächere elektrostatische Wechselwirkungen, z.B. Wasserstoffbrückenbindungen. Das Schichtgefüge indessen ist weitgehend durch starke, kovalente Bindungen geprägt.

Die Stöchiometrie der Blattsilicate ist (Si₂O₅²⁻) für reine Silicatstrukturen und (AlₘSi₂₋ₘO₅^{(2+m)-}) für Alumosilicate.

m ist eine reale Zahl größer als Null und kleiner als 2.

Liegen keine reinen Silicate sondern Alumosilicate vor, ist dem Umstande Rechnung zu tragen, daß jede durch Al³⁺ ersetzte Si⁴⁺ - Gruppe ein weiteres einfach geladenes Kation zur Ladungsneutralisierung erfordert.

Die Ladungsbilanz wird bevorzugt durch H⁺, Alkali- oder Erdalkalimetallionen ausgeglichen. Auch Aluminium als Gegenion ist bekannt und vorteilhaft. Im Gegensatz zu den Alumosilicaten werden diese Verbindungen Aluminiumsilicate genannt. Auch "Aluminiumalumosilicate"_{,} in welchen Aluminium sowohl im Silicatnetz, als auch als Gegenion vorliegt, sind bekannt und für die vorliegende Erfindung gegebenenfalls von Vorteil.

Schichtsilicate sind in der Literatur gut dokumentiert, z.B. im "Lehrbuch der Anorganischen Chemie", A.F. Hollemann, E. Wiberg und N. Wiberg, 91.-100. Aufl., Walter de Gruyter - Verlag 1985, passim, sowie "Lehrbuch der Anorganischen Chemie", H.Remy, 12. Aufl., Akademische Verlagsgesellschaft, Leipzig 1965, passim. Die Schichtenstruktur von Montmorillonit ist Römpps Chemie-Lexikon, Franckh'sche Verlagshandlung W. Keller & Co., Stuttgart, 8.Aufl., 1985, S. 2668 f., zu entnehmen.

Beispiele für Schichtsilicate sind:
- Montmorillonit oft vereinfacht:: Na_{0,33}((Al_{1,67}Mg_{0,33})(OH)₂(Si₄O₁₀))
Al₂O₃*4SiO₂*H₂O*nH₂O
- Kaolinit: Al₂(OH)₄(Si₂O₅)
- Ilit: (K,H₃O)_{y}(Mg₃(OH)₂(Si_{4-y}Al_{y}O₁₀)) und
(K,H₃O)_{y}(Al₂(OH)₂(Si_{4-y}Al_{y}O₁₀))
mit y = 0,7 - 0,9
- Beidellit: (Ca,Na)_{0,3}(Al₂(OH)₂(Al_{0,5}Si_{3,5}O₁₀))
- Nontronit: Na_{0,33}(Fe₂(OH)₂(Al_{0,33}Si_{3,67}O₁₀))
- Saponit: (Ca,Na)_{0,33}((Mg,Fe)₃(OH)₂(Al_{0,33}Si_{3,67}O₁₀))
- Hectorit: Na_{0,33}((Mg,Li)₃(OH,F)₂(Si₄O₁₀))
Montmorillonit stellt das Hauptmineral der natürlich vorkommenden Bentonite dar.

Die Wirkung der erfindungsgemäßen Zusammensetzungen beruht möglicherweise darauf, daß die Schichtsilicate flüchtige Substanzen absorbieren, die bei der mikrobiellen Zersetzung des frischen Schweißes auf der Haut entstehen. Unangenehmer Schweißgeruch wird verhindert. Die Mikroflora der Haut bleibt gänzlich unbeeinträchtigt.

ES war erstaunlich, daß die erfindungsgemäßen Zusammensetzungen nicht nur für kosmetische Zwecke geeignet sind, sondern überdies wirkungsvoller und schonender sind als die Zusammensetzungen des Standes der Technik.

Es ist zwar bekannt, organisch modifizierte Tone, auch Bentonite, in kosmetischen Formulierungen einzusetzen. Bekannt sind ferner kosmetische Desodorantien mit einem Gehalt an solchen modifizierten Tonen, z.B. EP-0 319 168. Diese Stoffe indes sind gemäß ihrer chemischen Natur völlig ungeeignet, als desodorierende Agentien zu wirken. Sie stellen in den Formulierungen des Standes der Technik nur Hilfs- oder Zusatzstoffe dar, die die Konsistenz der Formulierungen verbessern sollen oder ähnliches.

Aus FR-A-1 199 349 ist ferner ein Rasierpuder mit einem Gehalt an Bentonit und smektischem Ton bekannt, welcher angeblich desodorierende Eigenschaften habe. Eine solche Zusammensetzung ist aber gänzlich ungeeignet, durch Zersetzung apokrinen Schweißes hervorgerufenen Körpergeruch zu beseitigen oder zu verhindern.

Es hat sich herausgestellt, daß sich Schichtsilicate erfolgreich in alle gewöhnlichen Formulierungstypen von Desodorantien einarbeiten lassen, beispielsweise in Aerosole, Pumpsprays, Pudersprays, Roll-ons, Deostifte, Tinkturen und weitere mehr.

Die Schichtsilicate, welche Aluminium in ihrem Gerüst tragen oder Aluminium als Gegenionen haben, also Alumosilicate und Aluminiumsilicate, sind in den erfindungsgemäßen Zusammensetzungen besonders bevorzugt.

Es ist selbverständlich, daß als Gegenkationen nur solche in Frage kommen, die, topisch angewandt, gesundheitsverträglich sind. Weiterhin ist selbstverständlich, daß native Mineralien so aufbereitet werden müssen, daß sie kosmetische Ansprüche erfüllen. Unerheblich ist hingegen, ob die Schichtsilicate synthetisch erzeugt oder aus nativem Material gewonnen wurden. Schichtsilicate, die der Zusammensetzung eines natürlichen Schichtsilicates entsprechen, sind gleichermaßen vorteilhaft.

Vorzugsweise liegen die Schichtsilicate in Konzentrationen von 1,0 - 40,0 Gew.-% vor. Besonders bevorzugt liegen die Schichtsilicate in Konzentrationen von 5,0 - 20,0 Gew.-%, ganz besonders bevorzugt in Konzentrationen von 7,5 - 15,0 Gew.-% vor. Die Konzentrationsangaben beziehen sich jeweils auf das Gesamtgewicht der Zusammensetzung.

Als besonders vorteilhaft haben sich Formulierungen mit einem wirksamen Gehalt an Bentoniten, Smectiten, sowie reinem Montmorillonit herausgestellt.

Es ist möglich und gegebenenfalls vorteilhaft, ein Gemisch der verschiedenen Schichtsilicate als wirksames Prinzip der erfindungsgemäßen Zusammensetzungen einzusetzen.

Günstig ist, die Menge an polaren Substanzen so niedrig zu halten wie möglich. Ethanol, in einigen Formulierungen unverzichtbar, sollte nicht in Konzentrationen höher als 35 Gew.-% vorliegen.

Die erfindungsgemäßen Zusammensetzungen sollten weitgehend wasserfrei sein. Wasser bindet freie Koordinationsstellen der Schichtsilicate und vermindert deren Wirksamkeit. Der Wassergehalt der erfindungsgemäßen Zusammensetzungen sollte daher 5,0 Gew.-%, bezogen auf die gesamte Zusammensetzung, nicht überschreiten. Die Wirksamkeit der Zusammensetzungen ist dann aber schon stark beeinträchtigt.

Es kann ferner von Vorteil sein, den Zusammensetzungen die üblichen kosmetischen Zusatzstoffe einzuverleiben, beispielsweise Konservierungsstoffe, Antioxidantien, Photostabilisatoren usw.

Ansonsten sind die üblichen Maßregeln für das Zusammenstellen von kosmetischen Formulierungen zu beachten, die dem Fachmann geläufig sind.

Die Schichtsilicate können auf einfache Weise in die erfindungsgemäßen Zusammensetzungen eingearbeitet werden. Bevorzugt werden sie als feinstzerteilte Partikel den übrigen Bestandteilen der Formulierungen zugesetzt, vorteilhaft in Gegenwart eines Dispergiermittels.

Die folgenden Beispiele dienen dazu, vorteilhafte Verkörperungen der vorliegenden Erfindung zu beschreiben, ohne daß beabsichtigt ist, die Erfindung auf diese Beispiele zu beschränken.

### Beispiel 1

| Roll-on-Formulierung | |
|---|---|
| | Gew.-% |
| Bentonit EX 0105 | 10,0 |
| Isohexadecan | 64,5 |
| C₁₂₋₁₅-Benzoat | 10,0 |
| Tixogel Mio | 15,0 |
| Parfumöl | 0,5 |

### Beispiel 2

| Deo- / Antitranspirantspray | |
|---|---|
| | Gew.-% |
| Volatile Silicone | 36,0 |
| Isopropylpalmitat | 5,0 |
| Ethanol (absolut) | 25,0 |
| Aerosil 200 | 2,0 |
| Bentonit EX 0105 | 30,0 |
| Parfumöl | 2,0 |

Die vorstehende Mischung wird mit der vierfachen Menge eines Gemisches Propan/Butan 2.7 zum Aerosolspray aufgefüllt.

### Beispiel 3

| Deo- / Antitranspirant- Stift | |
|---|---|
| | Gew.-% |
| Isopropyladipat | 20,0 |
| C₁₂₋₁₅-Benzoat | 15,0 |
| Stearylalkohol | 15,0 |
| Bienenwachs | 5,0 |
| Glycerinmonolaurat | 5,0 |
| Talkum | 29,5 |
| Bentonit EX 0105 | 10,0 |
| Parfumöl | 0,5 |

## Patentansprüche

1. Kosmetische Desodorantien in Form von Suspensionen,
(a) enthaltend ein oder mehrere Schichtsilicate, gewählt aus der Gruppe Montmorillonit, Kaolinit, Ilit, Beidellit, Nontronit, Saponit, Hectorit, Bentonit, Smectit,
(b) wobei der Wassergehalt der kosmetischen Desodorantien 5 Gew.-% nicht übersteigt,
(c) wobei der Ethanolgehalt der kosmetischen Desodorantien 35 Gew.-% nicht übersteigt.

2. Kosmetische Desodorantien nach Anspruch 1, dadurch gekennzeichnet, daß das oder die Schichtsilicate in Konzentrationen von 1,0 - 40,0 Gew.-% , bezogen auf das Gesamtgewicht der Zusammensetzung, vorliegen.

3. Kosmetische Desodorantien nach Anspruch 1, dadurch gekennzeichnet, daß das oder die Schichtsilicate in Konzentrationen von 5,0 - 20,0 Gew.-% , bezogen auf das Gesamtgewicht der Zusammensetzung, vorliegen.

4. Kosmetische Desodorantien nach Anspruch 1, dadurch gekennzeichnet, daß das oder die Schichtsilicate in Konzentrationen von 7,5 - 15,0 Gew.-% , bezogen auf das Gesamtgewicht der Zusammensetzung, vorliegen.

5. Kosmetische Desodorantien nach Anspruch 1, dadurch gekennzeichnet, daß sie in Form von Aerosolen, Roll-ons, Pumpsprays, Puder, Pudersprays, Deostifte oder Tinkturen vorliegen.

## Claims

1. Cosmetic deodorants in the form of suspensions
(a) containing one or more sheet silicates selected from the group consisting of montmorillonite, kaolinite, ilite, beidellite, nontronite, saponite, hectorite, bentonite, smectite,
(b) where the water content of the cosmetic deodorants does not exceed 5% by weight,
(c) where the ethanol content of the cosmetic deodorants does not exceed 35% by weight.

2. Cosmetic deodorants according to Claim 1, characterized in that the sheet silicate(s) is/are present in concentrations of 1.0 - 40.0% by weight, based on the total weight of the composition.

3. Cosmetic deodorants according to Claim 1, characterized in that the sheet silicate(s) is/are present in concentrations of 5.0 - 20.0% by weight, based on the total weight of the composition.

4. Cosmetic deodorants according to Claim 1, characterized in that the sheet silicate(s) is/are present in concentrations of 7.5 - 15.0% by weight, based on the total weight of the composition.

5. Cosmetic deodorants according to Claim 1, characterized in that they are present in the form of aerosols, roll-ons, pump sprays, powder, powder sprays, deodorant sticks or tinctures.

## Revendications

1. Agents cosmétiques désodorisants sous forme de suspensions,
(a) contenant un ou plusieurs silicates stratifiés, choisis parmi le groupe
montmorillonite, kaolinite, illite, beidellite, nontronite, saponite, hectorite, bentonite, smectite,
(b) la teneur en eau des agents cosmétiques désodorisants ne dépassant pas 5 % en poids,
(c) la teneur en éthanol des agents cosmétiques désodorisants ne dépassant pas 35 % en poids.

2. Agents cosmétiques désodorisants selon la revendication 1, caractérisés en ce que le ou les silicates stratifiés sont présents dans des concentrations de 1,0 - 40,0 % en poids par rapport au poids total de la composition.

3. Agents cosmétiques désodorisants selon la revendication 1, caractérisés en ce que le ou les silicates stratifiés sont présents dans des concentrations de 5,0 - 20,0 % en poids par rapport au poids total de la composition.

4. Agents cosmétiques désodorisants selon la revendication 1, caractérisés en ce que le ou les silicates stratifiés sont présents dans des concentrations de 7,5 - 15,0 % en poids par rapport au poids total de la composition.

5. Agents cosmétiques désodorisants selon la revendication 1, caractérisés en ce qu'ils sont présents sous forme d'aérosols, de produits à application "roll-on", de sprays pour pompage, de poudres, des sprays en poudre, de bâtons désodorisants ou de teintures.
